# EUROPEAN PATENT SPECIFICATION

(11) **EP 1 948 098 B1**
(45) Date of publication and mention of the grant of the patent: **07.05.2014**
(21) Application number: 06809846.6
(22) Date of filing: 07.11.2006
(51) Int. Cl.: A61F 2/82

(54) **BIFURCATED STENT ASSEMBLY**
GEGABELTE STENTANORDNUNG
ENSEMBLE ENDOPROTHESE A BIFURCATION

(30) Priority: 07.11.2005 US 267311
(43) Date of publication of application: 30.07.2008
(73) Proprietor: DESIGN & PERFORMANCE - CYPRUS LIMITED, Nicosia, 1066 (CY)
(72) Inventor: VONDERWALDE, Carlos, Richmond, British Columbia V7C 4P8 (CA)
(74) Representative: Kurig, Thomas
(86) International application number: PCT/IL2006/001286
(87) International publication number: WO 2007/052280

(56) References cited:
- WO-A1-99/13808
- DE-A1- 19 533 589
- US-A- 5 575 817
- US-A- 5 643 208
- US-A- 6 102 938
- US-A1- 2003 199 967

## Description

### FIELD AND BACKGROUND OF THE INVENTION

The present invention relates to the field of surgery and more particularly, to a method and a device useful for maintaining patency of a bifurcated lumen, especially of the cardiovascular system.

A stent is a device deployed inside a lumen of a bodily vessel to physically maintain patency of the vessel. Typical vessels treated with stents include respiratory ducts, gastrointestinal ducts, lymphatic ducts, blood vessels and especially arteries that are occluded, stenosed, aneuritic, physically damaged, diseased, collapsing or weakened.

Stents are usually outwardly radially expandable, having a substantially tubular shape both in an unexpanded state with a small radial dimension and in any one of the expanded states with larger radial dimensions. Various constructions of stents are known including rolled-up sheets, slotted or otherwise cut-out tubes and bent wires.

For deployment inside a lumen of a bodily vessel an expandable stent is placed in an unexpanded state on a deployment catheter, inserted through an incision in the skin and maneuvered through the body to the deployment location. The stent is then expanded to an appropriately-sized expanded state so as to engage the inner walls of the treated vessel and to thus maintain patency thereof.

A first type of stent is the self-expanding stent. When the stent is at the deployment location, the stent is released from the catheter and allowed to expand to an expanded state, in a manner analogous to that of a compressed spring. Self-expanding stents have been disclosed, for example, in U.S. Patent Nos. 4,503,569; 4,580,568; 4,787,899; and 5,104,399.

A second type of stent is expanded from the unexpanded state to an expanded state using an expansion device, typically a catheter-borne balloon. When the stent is at the deployment location, the expansion device is activated inside the bore of the unexpanded stent to exert an outwards radial force on the inside of the stent, causing the stent to expand to a desired size. Such stents have been disclosed, for example, in U.S. Patents Nos. 4,655,771; 4,733,665; 4,739,762; 4,800,882; 4,907,336; 4,994,071; 5,019,090; 5,035,706; 5,037,392; and 5,147,385.

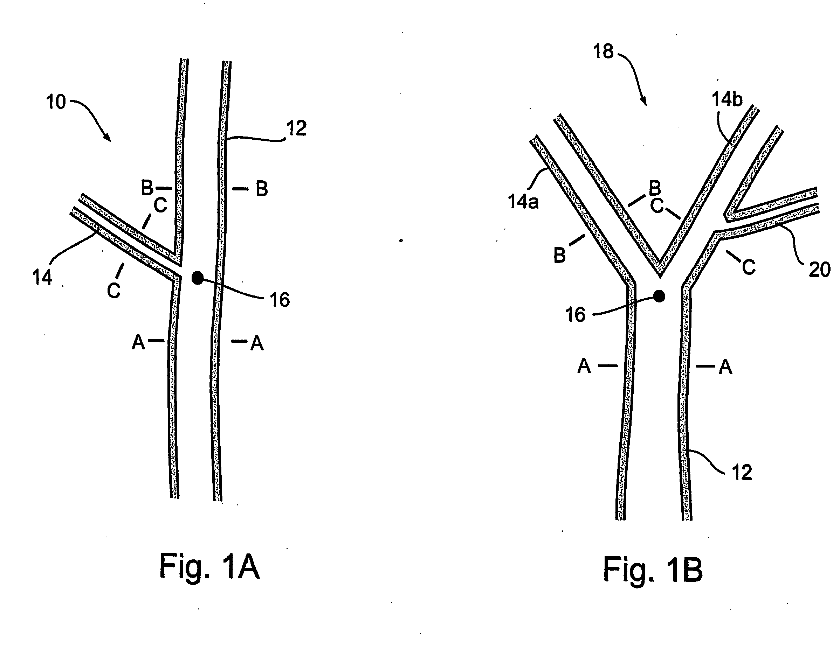

As is known to one skilled in the art, many bodily vessels are bifurcated. By "bifurcated" is meant an object that splits to two branches along a length of the object. Two types of bifurcated objects (bifurcated blood vessels) having two different types of bifurcation are depicted in Figures 1A and 1B. A first type of bifurcated object **10** depicted in Figure 1A includes a trunk vessel **12** from which a branch vessel **14** branches downstream from a bifurcation point **16.** Generally, but not necessarily, the bore of branch vessel **14** is smaller than that of trunk vessel **12.** A second type of bifurcated object **18** depicted in Figure 1B includes a trunk vessel **12** from which two branch vessels **14A** and **14B** branch downstream from bifurcation point **16.** Generally, but not necessarily, the bores of branch vessels **14A** and **14B** are smaller than the bore of trunk vessel **12.** Branch vessel **14B** of bifurcated object **18** is also bifurcated: branch vessel **14B** is a trunk vessel from which branch vessel **20** branches. Although many naturally occuring vessels can be clearly classified as having either the first or the second type of bifurcation there are cases where such classification is ambiguous as there is no exact delineation of characteristics distinguishing one of the two types of bifurcation from the other.

In the field of medicine it is known to deploy stents in a bifurcated bodily vessel. Often, both branches of a bifurcated bodily vessel must be treated as damage, lesions or athersclerotic disease are often found in both the trunk and the branch vessels. However, even in cases where a branch vessel is healthy, interventional manipulation of the trunk vessel often compromises the integrity of the branch vessel. It is therefore preferable to deploy more than one stent in a bifurcated vessel, see for example U.S. Patent Nos. 4,994,071; 5,669,924; 5,723,004; 5,906,640 or PCT patent application No. PCT/IB98/00496 published as WO 99/15103 of the inventor.

The challenges in deploying stents in a bifurcated vessel are generally a result of the fact that the component vessels of the bifurcated vessel are of different sizes and that the component vessels or the vicinity of the bifurcation point are often not flexible, structurally weak and susceptible to tearing due to damage, disease or age. Ideally:
a) each individual stent is expanded independently to an appropriate extent to provide a bore of the desired size to the branch or trunk vessel in which deployed;
b) care is taken when expanding a stent in a larger branch or trunk vessel not to damage or cause catastrophic failure of a proximate smaller branch vessel or in the vicinity of the bifurcation point;
c) the stents are mechanically uncoupled so that movement, orientation and expansion of each individual stent is completely free and manipulation of one stent does not influence or effect another. This freedom of motion is preferably to a great extent as possible allowing the greatest possible of variation of divergence angle and relative orientation of one stent to another;
d) there is no pinching of bodily parts between any two stents;
e) the vessel is physically supported in the vicinity of the bifurcation point to prevent collapse of the vessel at the bifurcation point, but without excess force that may lead to rupture of the bifurcation point;
f) care is taken to ensure that flow through the treated vessels and the bifurcation point is unimpeded and not turbulent; and
g) once deployed the orientation between the two stents can change together with the natural movement of the organ (e.g., beating of the heart) in which deployed to prevent structural stress of the bifurcated vessel.

Strategies for treating a bifurcated vessel by deploying multiple independent stents in each branch or trunk vessel are summarized in U.S. Patent No. 5,669,924. In a bifurcated blood **vessel 10** as depicted in Figure 1A, a branch-supporting stent is deployed in branch vessel **14** from C-C to the beginning of branch vessel **14** and subsequently a trunk-supporting stent is deployed in trunk vessel **12** both upstream and downstream of bifurcation point **16** from A-A to B-B. In a bifurcated blood vessel such as **18** as depicted in Figure 1B, three individual stents are deployed, one branch-supporting stents in each of branch vessels **14A** (from the bifurcation to B-B) and **14B** (from the bifurcation to C-C) as well as a trunk-supporting stent in trunk vessel **12** (from the bifurcation to A-A).

Deployment of separate stents is problematic for a number of reasons. For support in the vicinity of the bifurcation point **16** it is necessary to deploy the stents close to bifurcation point **16,** yet this gives no support to the immediate vicinity of bifurcation point 16. In some cases, one end of branch-supporting stent deployed in branch vessel **14** protrudes into trunk vessel **12,** leading to turbulent flow through trunk vessel **12** and into branch vessel **14,** (increasing the chances for restenosis) or making it impossible to deploy a trunk-supporting stent in trunk vessel **12.** Further, if the stents are too close together, one stent may tangle with another or parts of the blood vessels may be pinched between the two stents. Expansion of multiple stents that are very close one to the other can lead to extreme stress and tearing of the blood vessels. To avoid such problems, the stents must be deployed far from each other, creating a large unsupported area in the vicinity of bifurcation point **16.** Additionally, when treating vessels such as a bifurcated blood vessel **10** in Figure 1A, a wall of a trunk-supporting stent deployed in trunk vessel 12 between A-A and B-B necessarily interferes with flow into branch vessel **14.**

Another strategy discussed in U.S. Patent No. 5,669,924 is the deployment of a stent assembly made up of two interleaved stents in blood vessels such as bifurcated blood vessel **18** depicted in Figure 1B. In such a stent assembly, a downstream end of a first stent is deployed in branch vessel **14A,** a downstream end of a second stent is deployed in branch vessel **14B** and both upstream ends are interleaved and deployed in trunk vessel **12.** Such a strategy is inadequate for a number of reasons. Since the ends of the stents deployed in branch vessels **14A** and **14B** offer less resistance to an expansion device than do the interleaved ends deployed in trunk vessel **12,** the parts of stents in branch vessels **14A** and **14B** are expanded to a greater extent than the parts of the stents deployed in trunk vessel **12,** even though trunk vessel **12** generally has a larger bore than do branch vessels **14A** and **14B.** Further, only certain types of stents are interleavable, limiting the applicability of such a strategy. Further, the interleaving of the two stents generally interferes with the proper expansion of the stents, leading to suboptimal support of vessel **18** and turbulent flow therethrough. Further, parts of vessel **18** may be pinched between the stents.

A number of bifurcated stent assemblies, generally comprising two or more conjoined stents are known in the art. Herein the freedom of independent motion of the conjoined stents, is termed bendability. The term in-plane bendability refers to independence of motion of any two stents within the plane defined by the axes of the two stents, especially with regards to the angle of divergence of the stents. The term out-of-plane bendability refers to independence of motion of any two stents outside of the plane defined by the axes of the two stents. A stent assembly having a high degree of bendability is one where the motion of a first stent of a stent assembly relative to a second stent of the assembly is possible to a high degree without bending, distorting or buckling of the second stent.

In U.S. Patent No. 4,994,071 is taught a bifurcated stent assembly substantially comprising a single trunk-supporting stent having two axial backbone wires with two branch-supporting stents connected to the trunk-supporting stent, each branch-supporting stent to one of the two axial backbone wires. The stent assembly of U.S. Patent No. 4,994,071 is suitable for deployment in vessels similar to vessel **18** depicted in Figure 1B but no embodiment suitable for deployment in vessel **10** depicted in Figure 1A is disclosed. A disadvantage of the stent assembly of U.S. Patent No. 4,994,071 is that little support is given to the vicinity of a bifurcation point **16.** A further disadvantage is that the structure of the stent assembly allows for pinching of parts of the bodily vessel between the two branch-supporting stents. A further disadvantage is that the bendability of the stent assembly is low as the component stents are mechanically coupled through the axial backbone wires. A further disadvantage is that the bendability of the stent assembly is low as the bendability arises from the flexibility of the axial backbone wires which is homogenous along the length of a given wire. As a result, a bending force applied to one stent flexes that stent rather than changing the relative orientation of two component stents.

In order to overcome some of the disadvantages of the bifurcated expandable stent assembly of U.S. Patent No. 4,994,071, U.S. Patent No. 5,723,004 teaches a bifurcated expandable graft secured to the bifurcated stent assembly of U.S. Patent No. 4,994,071. The graft is made up of a flexible liner and/or a flexible jacket applied to the stent assembly. The graft prevents pinching between the component stents and provides a matrix for cell growth so that ultimately the vicinity of the bifurcation growth is mechanically strengthened by the new cells. The graft allegedly provides support to the vicinity of the bifurcation point. That said, the liner / jacket does not have a structural role in the stent assembly which is provided exclusively by the stents. As a result, the component stents must be associated through axial backbone wires as described above so the limited bendability and the mechanical coupling of the stents remain. Further, in U.S. Patent No. 5,723,004 the expensive and complex methods disclosed for producing the stent assembly limit the utility of the teachings: a liner is applied to a mandrel (e.g., by electrospinning, dipping or fiber winding of synthetic materials), a stent is crimped over the liner on the mandrel and subsequently a jacket is applied over the stent held on the mandrel. Further, the teachings of U.S. Patent No. 5,723,004 are directed exclusively to permeable synthetic grafts to allow cell growth therethrough. No solid, non-permeable graft is disclosed nor is any use of non-synthetic materials suggested. Further, the thickness of the graft is not discussed but, as is seen in the figures is quite significant: such a thick graft either leads to the stent assembly having a small bore and thus forming a flow bottleneck once deployed, or requires over-expansion which may cause the vessel in which the stent assembly is deployed to rupture.

In PCT patent application No. PCT/IB98/00496 published as WO 99/15103 of the inventor is taught an articulated stent assembly. To the side of a trunk stent is attached a branch stent by some method or mechanism that substantially defines an articulation or joint, e.g., a hinge. For deployment, the divergence angle of the branch stent relative to the trunk stent is varied as desired at the joint with substantially no distortion or buckling of either stent. The close coupling of the component stents ensures that support is given to the bifurcation point. A disadvantage of the teachings of PCT patent application No. PCT/IB98/00496 is that not all stents may be suitable to be coupled using an articulation or joint. A device according to the preamble of claim 2 is known from the document WO-A-99/13808.

It would be highly advantageous to have a bifurcated stent assembly useful for deployment in bifurcated vessels and not having at least some of the disadvantages of the prior art.

### SUMMARY OF THE INVENTION

The present invention successfully addresses at least some of the shortcomings of the prior art by providing a method for bendably associating two stents and a bifurcated stent assembly for deployment in a bifurcated bodily vessel.

The present invention is based in the use of a flexible membrane, preferably a tubular membrane, as a stent connector to bendably hold and functionally associate two stents that are components of a bifurcated stent assembly. The flexible stent connector of the present invention allows the relative orientation of the two stents to be varied with little if any deformation of the connected stents. In preferred embodiments, the stent connector is configured to provide support to the bodily vessel in the vicinity of the bifurcation and to prevent pinching of bodily parts between the two stents.

According to the teachings of the present invention there is provided a method for bendably associating a first expandable stent with a second expandable stent for deployment in a bifurcated vessel comprising using a flexible membrane as a stent connector to connect the second expandable stent to the first expandable stent so that the bore of the first expandable stent defines an elongated trunk volume and the bore of the second expandable stent defines an elongated branch volume branching from the trunk volume wherein the two stents are substantially devoid of direct physical association. In an embodiment of the present invention the two stents are also devoid of mutual physical contact.

In an embodiment of the present invention, the stent connector comprises at least one filament. By filament is meant that at least part of the stent connector has an elongated shape such as that of a band, filament, ribbon, strand, string, strip or thread.

In a preferred embodiment of the present invention, at least part of the stent connector is substantially tubular.

The stent connector includes a substantially tubular trunk part functionally asociated with the first expandable stent. The substantially tubular trunk part of the stent connector surrounds at least part of the first expandable stent. The substantially tubular trunk part of the stent connector is disposed within the bore of the first expandable stent.

The stent connector includes a substantially tubular branch part functionally associated with the second expandable stent. The substantially tubular branch part of the stent connector surrounds at least part of the second expandable stent. The substantially tubular branch part of the stent connector is disposed within the bore of the second expandable stent.

In an embodiment of the present invention the stent connector includes a first substantially tubular trunk part functionally associated with the first expandable stent and a second substantially tubular branch part functionally associated with the second expandable stent, where the branch part of the stent connector branches from the trunk part of the stent connector.

In an embodiment of the present invention the branch volume is in fluid communication with the trunk volume from a side of the trunk volume, preferably through an opening in the side of the first expandable stent.

In an embodiment of the present invention the branch volume is in fluid communication with the trunk volume from an end of the trunk volume.

According to the teachings of the present invention there is also provided a bifurcated stent assembly, comprising a) a flexible membrane as a stent connector; b) a first expandable stent functionally associated with the stent connector, wherein the bore of the first expandable stent defines an elongated trunk volume; and c) a second expandable stent functionally associated with the stent connector, wherein the bore of the second expandable stent defines an elongated branch volume branching from the trunk volume wherein the stents are substantially devoid of direct physical association. In an embodiment of the present invention the two stents are also devoid of mutual physical contact. In an embodiment of the present invention, the stent connector comprises at least one filament.

The branch volume is in fluid communication with the trunk volume from a side of the trunk volume. In an embodiment of the present invention, the first expandable stent is provided with a side opening through which the branch volume is in fluid communication with the trunk volume.

In an embodiment of the present invention the branch volume is in fluid communication with the trunk volume from an end of the trunk volume.

In an embodiment of the present invention one, some or all of the component stents of a bifurcated stent assembly of the present invention is secured to the stent connector by at least one member of the group consisting of sutures, adhesives, bending members, clamps, glue, hooks, piercing members and staples.

In a preferred embodiment of the present invention at least part of the stent connector of a bifurcated stent assembly of the present invention is substantially tubular.

The stent connector includes a substantially tubular trunk part functionally associated with the first expandable stent.

In an embodiment of the present invention, the substantially tubular trunk part of the stent connector surrounds at least part of the first expandable stent. In an embodiment of the present invention, the first expandable stent is substantially entirely contained within the stent connector, especially a tubular part thereof, especially the trunk part thereof. In an embodiment of the present invention, at least one and preferably both of the ends of the first expandable stent emerge from the stent connector.

The trunk part of the stent connector is disposed within the bore of the first expandable stent. In embodiments of the present invention, the first expandable stent is entirely outside the stent connector. In an embodiment of the present invention, the trunk part of the stent connector is substantially entirely disposed within the bore of the first expandable stent. In an embodiment of the present invention, either one or both ends of the substantially tubular trunk part of the stent connector emerge from an end of the first expandable stent, and preferably are folded over a respective end.

The stent connector includes a substantially tubular branch part functionally associated with the second expandable stent.

In an embodiment of the present invention, the branch part of the stent connector surrounds an end of the second expandable stent.

In an embodiment of the present invention, the second expandable stent is substantially entirely contained within the stent connector, especially a tubular part thereof, especially the branch part thereof. In an embodiment of the present invention, an end of the second expandable stent emerges from the stent connector.

The branch part of the stent connector is disposed within the bore of the second expandable stent. In an embodiment of the present invention, the second expandable stent is entirely outside the stent connector. In an embodiment of the present invention, the branch part of the stent connector is substantially entirely disposed within the bore of the second expandable stent. In an embodiment of the present invention, an end of the substantially tubular branch part of the stent connector emerges from an end of the second expandable stent, and is preferably folded over the respective end.

In a preferred embodiment of the present invention the stent connector includes at least two substantially tubular parts: a first substantially tubular trunk part functionally associated with the first expandable stent and a second substantially tubular branch part functionally associated with the second expandable stent, where the branch part branches from the trunk part. In a preferred embodiment of the present invention, the first expandable stent is provided with a side opening, the stent connector is at least partially disposed within the bore of the first expandable stent so that the branch part of the stent connector emerges from the side opening of the first expandable stent.

In embodiments of the present invention, a bifurcated stent assembly of the present invention is made up of more than two expandable stents. Generally, additional stents are all stents that, analogously to the second expandable stent, branch from the first expandable stent.

In an embodiment of the present invention, a bifurcated stent assembly of the present invention further comprises a third expandable stent functionally associated with the stent connector, wherein the bore of the third expandable stent defines an elongated second branch volume branching from the trunk volume. In an embodiment of the present invention, the second branch volume is in fluid communication with the trunk volume from a side of the trunk volume, preferably through an opening in the side of the first expandable stent. In an embodiment of the present invention the second branch volume is in fluid communication with the trunk volume from an end of the trunk volume. In an embodiment of the present invention, the stent connector includes a substantially tubular branch part functionally associated with the third expandable stent.

In an embodiment of the present invention, the stent connector is substantially a vessel comprising walls of a flexible material and at least three ports, a first port, a second port and a third port, and, wherein the first expandable stent is functionally associated with the first port and the second expandable stent is functionally associated with the third port. In an embodiment of the present invention, the first expandable stent is functionally associated with both the first port and the second port. In an embodiment of the present invention, the bifurcated stent assembly further comprises a third expandable stent functionally associated with the second port. In an embodiment of the present invention, the stent connector has exactly three ports.

In an embodiment of the present invention, the first port, the second port and the walls of the stent connector therebetween define a trunk part of the stent connector that encloses a volume that substantially overlaps the trunk volume of the stent assembly.

In an embodiment of the present invention, the first expandable stent is provided with a side opening. In an embodiment of the present invention, the side opening of the first expandable stent is functionally associated with the third port of the stent connector. In an embodiment of the present invention, the bore of the second expandable stent is in fluid communication with the bore of the first expandable stent through the side opening.

In an embodiment of the present invention, the first port, the second port, the walls of the stent connector therebetween together with the first expandable stent substantially define at least part of a trunk implant, that is the part of the bifurcated stent assembly that when used is deployed in the trunk vessel.

In an embodiment of the present invention, the third port is in fluid communication with the trunk volume through a substantially tubular branch of the stent connector. In an embodiment of the present invention, the third port, the walls of the substantially tubular branch of the stent connector together with the second expandable stent substantially define at least part of a branch implant, that is the part of the bifurcated stent assembly that when used is deployed in the branch vessel.

In an embodiment of the present invention, at least part of the tubular branch is contained within the bore of the second expandable stent. In an embodiment of the present invention, the tubular branch is substantially entirely contained within the bore of the second expandable stent. In an embodiment of the present invention, a part of the tubular branch emerges from an end of the second expandable stent and is preferably folded thereover.

In an embodiment of the present invention, the flexible membrane of the stent connector is substantially elastic.

In an embodiment of the present invention, the flexible membrane of the stent connector is substantially impermeable to fluids.

In an embodiment of the present invention, the flexible membrane of the stent connector is substantially impervious to tissue proliferation therethrough. Such imperviousness is useful in preventing tissue buildup on and through the membrane and prevents the migration of smooth muscle cells. Such an embodiment is useful for providing a treated bodily vessel with a new, undamaged, smooth lining that is substantially impervious to restenosis.

In an embodiment of the present invention, the flexible membrane of the stent connector is permeable. Such an embodiment is useful as the membrane permeability allows cells to grow into and through the membrane, making the membrane substantially part of the vessel in which deployed.

Generally, the walls of a stent connector of the present invention are as thin as possible to ensure that a respective bifurcated stent assembly be flexible, have a low profile during navigation to the deployment site and to restrict a vessel in which deployed as little as possible and at the same time the walls must be sufficiently elastic and strong to permit navigation and deployment without tearing. Clearly, the nature of the material from which a given stent connector is made determines in part the thickness of the walls. That said, a stent connector preferably has walls that are not thicker than about 0.75 mm, not thicker than about 0.45 mm, not thicker than about 0.25 mm, not thicker than about 0.20 mm, and even not thicker than about 0.05 mm.

In an embodiment of the present invention, the stent connector and especially the stent connector wall are substantially fashioned from a synthetic or polymeric material including but not limited to polytetrafluoroethylene, urethane, elastomer, polyamide (e.g., Nylon) and polyester (e.g., Dacron).

In an embodiment of the present invention, the stent connector and especially the stent connector wall are substantially fashioned from biological tissue including but not limited to autologous tissue, heterologous tissue, venous tissue, arterial tissue, serous tissue, pleura, peritoneum, pericardium and aortic leaflet. In a preferred embodiment, the flexible membrane is a section of bifurcated biological tissue such as a bifurcated blood vessel (including a bifurcation) that is harvested and treated so as to prepare a seamless one-piece stent connector. In an embodiment of the present invention, the material is harvested from a source selected from the group consisting of human sources and non-human animal sources, especially equine, porcine, bovine and human. In an embodiment of the present invention the material is thinned, where after harvesting one or more layers of the harvested tissue is removed, e.g., by scraping, shaving, slicing or skiving. In an embodiment of the present invention, the material (or components of the material such as collagen) is cross-linked, for example by treatment with a glutaraldehyde or a phosphate solution.

In an embodiment of the present invention, the stent connector is substantially fashioned from serous membrane including a serous tissue stratum and a basement tissue stratum, where the serous membrane is, for example, from porcine, bovine, equine or human serous membrane.

In a preferred embodiment of the present invention, the stent connector is substantially fashioned from a thinned serous membrane, where a harvested serous membrane (peritoneum, pericardium or pleural tissue especially porcine, bovine equine and human serous tissue) has been processed by removal of a layer of at least some of the associated basement tissue (and thus thinned), preferably removal of all the basement tissue, leaving only the serous tissue layer. Thus in an embodiment of the present invention, the stent connector comprises a thinned serous membrane including a serous tissue stratum and a basement tissue stratum, wherein the thinned serous membrane has been processed by removal of a layer of basement tissue from a harvested serous membrane. In an embodiment of the present invention only a layer of the basement tissue is removed. In an embodiment of the present invention, the thinned serous membrane is substantially serous tissue devoid of basement tissue. In an embodiment of the present invention, the material consists essentially of serous tissue.

When serous tissue is used to implement the teachings of the present invention it is usually preferred to orient the membrane so that the smooth serous strata is facing the fluid flow to reduce turbulent flow.

In embodiments of the present invention, one or more of the component stents of the bifurcated stent assembly of the present invention are jacketed, for example with any of the jackets known in the art.

In embodiments of the present invention, one or more of the component stents of the bifurcated stent assembly of the present invention are coated, for example with any of the stent coatings known in the art.

Two parameters used when selecting a stent for use in implementing the teachings of the present invention are the expanded and unexpanded diameters of the stent.

Generally it is important that the unexpanded diameter of a stent be as small as possible to ease navigation through the body to the deployment location. That said, the unexpanded diameter must be large enough to allow threading of the stent onto a deployment catheter and, for not-self expanding stents, a stent-expanding device such as a stent-expanding balloon. Although there may be some variation in the unexpanded diameter of even two identical stents depending on how the two stents are used, herein by unexpanded diameter is intended the outer diameter of an expandable stent when crimped to smallest practical diameter onto a delivery catheter for deployment.

In embodiments of the present invention, a first expandable stent, a second expandable stent (and third or more expandable stents if present) are of substantially similar or identical dimensions, especially length, expanded diameter and/or unexpanded diameter.

In embodiments of the present invention a first expandable stent, a second expandable stent (and third or more expandable stents if present) are of substantially different dimensions, especially length, expanded diameter and/or unexpanded diameter. In cases where the first expandable stent, the second expandable stent (and third or more expandable stents if present) are of different dimensions, generally the first expandable stent is larger (especially of larger expanded and/or unexpanded diameter) as it is generally the first expandable stent that is destined to be deployed in a trunk vessel rather than in a smaller-bored branch vessel.

In embodiments of the present invention, the diameter (expanded or unexpanded) of the first expandable stent is substantially similar to the respective diameter of the second expandable stent (and third or more expandable stents if present).

In embodiments of the present invention, the diameter (expanded or unexpanded) of the first expandable stent is larger than the respective diameter of the second expandable stent (and third or more expandable stents if present).

Generally, any given stent has a wide range of expanded diameters larger than a respective unexpanded diameter. The expanded diameter of a stent subsequent to deployment is determined by the user of the stent according to medical criteria including the natural size of the lumen of the vessel in which the stent is deployed. That said, self-expanding stents are characterized by a specific maximal expansion that is the maximal diameter of the stent when the stent is free from externally applied forces. Most non-self expanding stents are also characterized by a maximal expansion that is the greatest extent to which the stent is expandable without comprising the structural integrity thereof.

Generally, a first expandable stent and a second expandable stent of a bifurcated stent assembly of the present invention are relatively close together. The distance between the first expandable stent and the second expandable stent is no greater than about four, no greater than about three, no greater than about two, no greater than about one and even no greater than about half of an unexpanded diameter of the second expandable stent.

According to the teachings of the present invention there is also provided a method of preparing a bifurcated stent assembly of the present invention, generally comprising providing an appropriate stent connector and a required number of stents and assembling the components in accordance with the description and the figures herein. In embodiments of the present invention, tubular components of a stent connector are made of substantially tubular tissue harvested as is.

In embodiments of the present invention, a stent connector comprises conjoined tubular components. In embodiments of the present invention, one or more tubular components are seamless, for example of harvested tubular tissue. In embodiments of the present invention, bifurcated tubular components of a stent connector are made of harvested substantially bifurcated tubular tissue harvested. In embodiments of the present invention, tubular components of a stent connector are made of substantially planar sheet of material that is fashioned into a tube, for example by overlapping or abutting two edges of the sheet. In embodiments of the present invention, a tubular wall of a stent connector is made of a first material while other components are made of a second material.

In an embodiment of the present invention, a stent connector of the present invention is essentially fashioned from one sheet, preferably planar or substantially planar, of an appropriate membrane (synthetic or harvested) rolled up into shape (with abutting or overlapping edges) and then fixed in shape using any of the methods known in the art. Preferred shapes of sheets suitable for use in fashioning a stent connector from one sheet of material generally include shapes having at least one C2 symmetry axis and/or at least one external angle of no greater than 90° and/or at least 7 sides. Suitable shapes include cross shapes, "T"-shapes, "W"-shapes, "X"-shapes, "Y"-shapes and "Ψ"-shapes.

### BRIEF DESCRIPTION OF THE DRAWINGS

The invention is herein described, by way of example only, with reference to the accompanying drawings.
**FIGS. 1A** and **1B** are depictions of bifurcated objects in which deployment of a bifurcated stent assembly of the present invention is useful;
**FIGS. 2A** and **2B** are depictions of bifurcated stent assemblies including strip-like stent connectors;
**FIGS. 3A** and **3B** are depictions of bifurcated stent assemblies including stent connectors comprising a substantially tubular trunk part associated with a first expandable stent and a strip-like part associated with a second expandable stent;
**FIGS. 4A** and **4B** are depictions of bifurcated stent assemblies including stent connectors comprising a substantially tubular branch part associated with a second expandable stent and a strip-like part associated with a first expandable stent;
**FIGS. 5A, 5B, 5C** and **5D** are depictions of bifurcated stent assemblies of the present invention including stent connectors comprising a substantially tubular trunk part associated with a first expandable stent and a substantially tubular branch part associated with a second expandable stent;
**FIGS. 6A, 6B** and **6C** are depictions of bifurcated stent assemblies of the present invention including three expandable stents; and
**FIGS. 7A-7G** depict sheets of various shapes rolled-up and edges attached to fashion stent connectors
Figures 1A-1B, 2A-2B, 3A-3B, 4A-4B and 7A-G, do not disclose embodiments of the present invention.

### DESCRIPTION OF EMBODIMENTS OF THE INVENTION

The present invention is of a bifurcated stent assembly useful for deployment in bifurcated bodily vessels. The present invention is also of a method for bendably associating two stents.

The present invention is related to a bifurcated stent assembly useful for deployment in bifurcated bodily vessels including a trunk vessel and a branch vessel. The teachings of the present invention are based on the use of a flexible membrane, preferably a tubular membrane, as a stent connector to bendably hold and functionally associate two or more stents making up a bifurcated stent assembly of the present invention. Such a flexible stent connector allows two stents to be bendably associated so that the distance between the two stents is relatively fixed and the respective lumina are in unobstructed fluid communication but that the relative orientation of the axes of the stents is variable in virtually all directions with little deformation of the thus-associated stents.

In a preferred embodiment, the flexible membrane includes tubular sections that are associated with the substantially tubular component stents, provide support or relining of the vessel in which deployed, reduce turbulent flow in the vessel subsequent to deployment and, depending on the configuration, avoid or prevent pinching of the vessel interior during deployment.

Specifically, the teachings of the present invention relate to an innovative method of associating two expandable stents by using a flexible membrane as a stent connector. A stent assembly of the present invention comprises a stent connector functionally associated with a first expandable stent the bore of which defines an elongated trunk volume and a second expandable stent the bore of which defines an elongated branch volume, where the branch volume branches from the trunk volume.

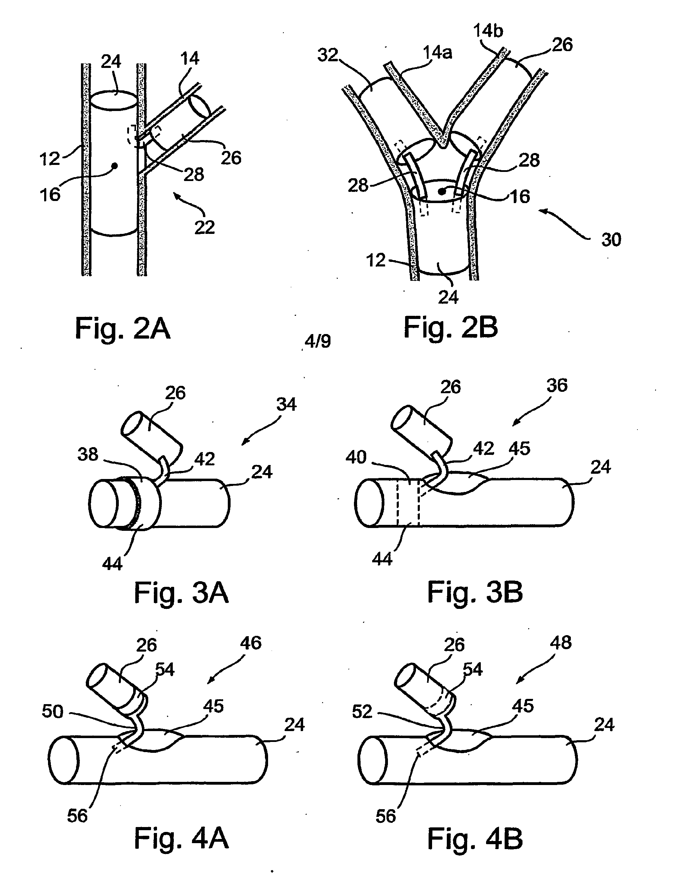

In Figures 2A and 2B are depicted two bifurcated stent assemblies where expandable stents are associated in accordance with the teachings of the present invention.

In Figure 2A, a bifurcated stent assembly **22** is made up of a first expandable stent **24,** a second expandable stent **26** and a stent connector **28** which is substantially a strip of heterologous tissue (*e.g*., serous tissue, such as from thinned equine pericardium). Stent assembly **22** may be deployed in a bifurcated artery such as **10** depicted in Fig 1A made up of a trunk artery **12** and a branch artery **14.** Stent connector **28** is secured to first expandable stent **24** and second expandable stent **26** by sutures (or optionally by sutures, adhesives, bending members, clamps, glue, hooks, piercing members, staples, other applicable mechanical mean or combinations thereof).

First expandable stent **24** is deployed in and supports patency of trunk artery **12** both upstream and downstream of bifurcation point **16** while second expandable stent **26** is deployed in and supports patency of branch artery **14.** The bore of first expandable stent **24** defines an elongated trunk volume and the bore of second expandable stent **26** defines an elongated branch volume branching from the trunk volume. As is seen in Figure 2A, the branch volume is in fluid communication with the trunk volume from the side of first expandable stent **24.**

Since first expandable stent **24** and second expandable stent **26** are devoid of mutual physical association or contact and attached only through stent connector **28,** manipulation and expansion of either stent during deployment of stent assembly **22** does not cause deformation such as buckling or stretching of the other stent.

In Figure 2B, a bifurcated stent assembly **30** is made up of a first expandable stent **24,** a second expandable stent **26,** a third expandable stent **32** and two stent connectors **28** which are substantially strips of heterologus tissue (*e.g*., serous tissue such as from thinned porcine pericardium). Stent assembly **30** is deployed in a bifurcated artery such as **18** depicted in Figure 1B made up of a trunk artery **12** that branches to two substantially similar sized branch arteries **14A** and **14B.** Stent connectors **28** are secured to first expandable stent **24,** second expandable stent 26 and third expandable stent **32** by sutures (or optionally by adhesives, bending members, clamp, glue, hooks, piercing members, staples, other applicable mechanical mean or combinations thereof). First expandable stent **24** is deployed in and supports patency of trunk artery **12** upstream of bifurcation point **16,** second expandable stent **26** is deployed in and supports patency of branch artery **14A** and third expandable stent **23** is deployed in and supports patency of branch artery **14B.** The bore of first expandable stent **24** defines an elongated trunk volume and the bores of second expandable stent **26** and third expandable stent **32** each define an elongated branch volume branching from the trunk volume. As is seen in Figure 2B the two branch volumes are in fluid communication with the trunk volume from an end of first expandable stent **24.**

Since first expandable stent **24,** second expandable stent 26 and third expandable stent **32** are devoid of mutual physical association or contact and attached only through stent connectors **26,** manipulation and expansion of any one stent during deployment of stent assembly **30** does not cause any deformation such as buckling or stretching in the other stents.

In Figures 2A and 2B, stent connectors **26** of bifurcated stent assemblies **22** and **30** are strips of heterologous tissue (*e.g*., serous tissue, pleura, peritoneum, pericardium), but in other examples are also bands, filaments, ribbons, strands, strings, strips or threads of any other suitable material.

In preferred embodiments of the present invention, at least part of a stent connector of a bifurcated stent assembly of the present invention is substantially tubular (including ring-shaped) allowing better association with one, two or more of the component tubular stents making up a given stent assembly. Better association includes such aspects as increased contact area between a stent and the respective tubular part of the stent connector reducing the chance of stent connector tearing, distribution of potentially deforming forces over a large area of a stent, better securing of the stent connector to an individual stent and more accurate relative positioning of any two stents.

The component stents of a stent assembly of the present invention are preferably associated with a tubular part of a stent connector. As is discussed in detail hereinbelow, examples of stent assemblies of the present invention made up of two stents have a stent connector with only one tubular part that one tubular part associated with a first stent or have a stent connector with only one tubular part that tubular part associated with a second stent. An embodiment of a stent assembly of the present invention has a stent connector with two tubular parts, a first tubular part associated with a first stent and a second tubular part associated with a second stent. Embodiments of stent assemblies of the present invention made up of three or more stents preferably have a stent connector comprising a tubular part associated with each one of the component stents.

In Figures 3A and 3B are depicted stent assemblies **34** and **36** respectively including stent connectors **38** and **40** respectively. Stent connectors **38** and **40** each have a strip-like branch part **42** associated with a second expandable stent **26** secured thereto by sutures (or optionally by adhesives, bending members, clamps, glue, hooks, piercing members, staples, other applicable mechanical mean or combinations thereof) analogously to stent connectors **28** of stent assemblies **22** and **30.** Stent connectors **38** and **40** each have a substantially tubular trunk part **44** (depicted in phantom in Figure 3B) associated with a first expandable stent **24.** In such a way, second expandable stent **26** is associated with first expandable stent **24** at a desired distance but with virtually no other limitations to the relative orientations of the two stents. Stent connectors **38** and **42** are made of a thinned layer of heterologous tissue preferably bovine pericardium (preferably thinned so as to consist essentially of serous tissue, see U.S. Patent Nos. 6,468,300 and 6,254,627 of the inventor) known for strength, tear-resistance, biocompatibility, expandability and elasticity. In both cases first expandable stent **24** is destined for deployment in a trunk artery while second expandable stent **26** is destined for deployment in a branch artery.

In Figure 3A, tubular trunk part **44** of stent connector **38** is disposed entirely on the outside of and surrounding first expandable stent **24,** and is held in place by tension and the attendant friction. Tubular trunk part **44** expands together with first expandable stent **24** during expansion thereof. Strip-like branch part **42** of stent connector 38 is disposed entirely on the inside of the bore of second expandable stent **26.**

In Figure 3B, first expandable stent **24** is provided with a side opening **45.** Tubular trunk part **44** of stent connector **40** is disposed entirely on the inside the bore of and surrounded by first expandable stent **24,** and is held in place by sutures (or optionally by adhesives, bending members, clamps, glue, hooks, piercing members, staples, other applicable mechanical mean or combinations thereof). Tubular trunk part **44** expands together with first expandable stent **24** during expansion thereof. Strip-like branch part **42** of stent connector **40** emerges from side opening **45** and is disposed entirely on the outside of second expandable stent **26.**

In Figures 4A and 4B are depicted stent assemblies **46** and **48** respectively including stent connectors **50** and **52** respectively. Stent connectors **50** and **52** each have a strip-like trunk part **56** associated with a first expandable stent **24** and secured thereto by sutures (or optionally by adhesives, bending members, clamps, glue, hooks, piercing members, staples, other applicable mechanical mean or combinations thereof) analogously to stent connectors **28** of stent assemblies **22** and **30.** Stent connectors **50** and **52** each have a substantially tubular branch part **54** (depicted in phantom in Figure 4B) associated with a second expandable stent **26.** In such a way, second expandable stent **26** is associated with first expandable stent **24** at a desired distance but with virtually no other limitations to the relative orientations of the two stents. Stent connectors **50** and **52** are made of a thinned layer of heterologous tissue preferably bovine pericardium (preferably thinned so as to consist essentially of serous tissue, see U.S. Patent Nos. 6,468,300 and 6,254,627 of the inventor) known for strength, tear-resistance, biocompatibility, expandability and elasticity. In both cases first expandable stent **24** is destined for deployment in a trunk artery while second expandable stent **26** is destined for deployment in a branch artery.

In Figure 4A, first expandable stent 24 is provided with a side opening **45.** Tubular branch part **54** of stent connector **50** is disposed entirely on the outside of and surrounding second expandable stent **26,** and is held in place by tension and the attendant friction. Tubular branch part **54** expands together with second expandable stent **26** during expansion thereof. Strip-like trunk part **56** of stent connector **50** passes through side opening **45** of first expandable stent **24** and is disposed entirely inside the bore of first expandable stent **24.**

In Figure 4B, first expandable stent **24** is provided with a side opening **45.** Tubular branch part **54** of stent connector **36** is disposed entirely inside the bore of and is substantially contained within second expandable stent **26,** and is held in place by sutures (or optionally by adhesives, bending members, clamps, glue, hooks, piercing members, staples, other applicable mechanical mean or combinations thereof). Tubular branch part **54** expands together with second expandable stent **24** during expansion thereof. Strip-like trunk part **56** of stent connector **52** is disposed entirely on the outside of first expandable stent **24.**

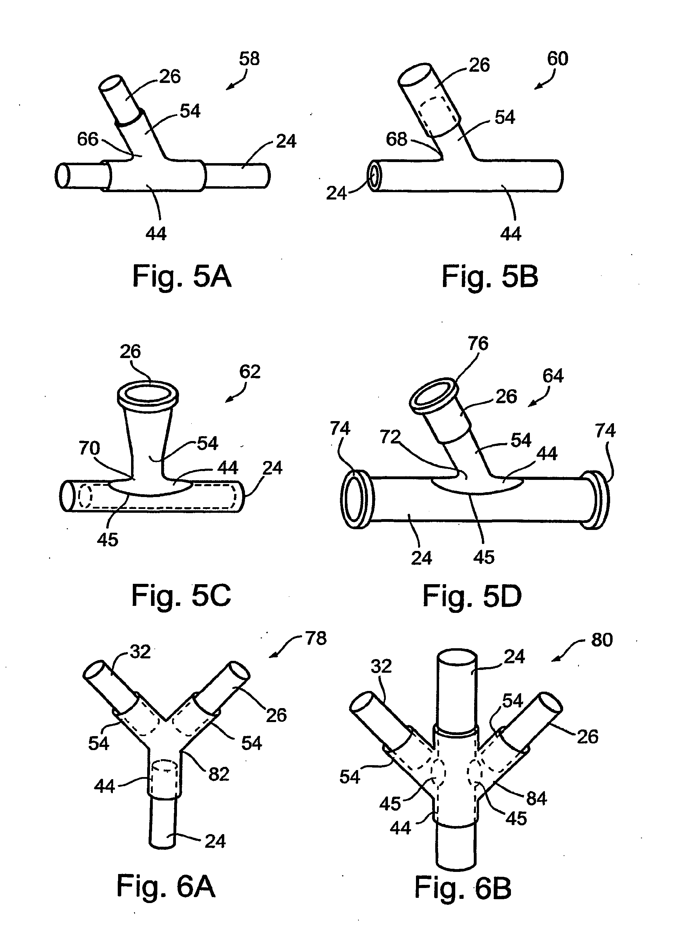

In Figures 5A, 5B, 5C and 5D are depicted stent assemblies of the present invention, **58, 60, 62** and **64** respectively including stent connectors **66, 68, 70** and **72** respectively. Stent connectors **66, 68, 70** and **72** each have two substantially tubular parts, a tubular trunk part **44** associated with a first expandable stent **24** and a tubular branch part **54** branching from trunk part **44** and associated with a second expandable stent **26.** In such a way, second expandable stent **26** is associated with first expandable stent **24** at a desired distance but with virtually no other limitations to the relative orientations of the two stents. When a tubular part **44** or **54** is disposed inside the bore of a respective stent, the tubular part is secured thereto by sutures (or optionally by adhesives, bending members, clamps, glue, hooks, piercing members, staples, other applicable mechanical mean or combinations thereof). When a tubular part **44** or **54** is disposed on the outside of a respective stent, the tubular part is preferably held in place by tension and the attendant friction (although in embodiments of the present invention the tubular part is held in place by sutures, adhesives, bending members, clamps, glue, hooks, piercing members, staples, other applicable mechanical mean or combinations thereof). A given tubular part expands together with a respective stent during expansion thereof.

In Figure 5A, tubular trunk part **44** of stent connector **66** is on the outside of and surrounds part of first expandable stent **24.** The two ends of first expandable stent **24** emerge from tubular trunk part **44.** Tubular branch part **54** of stent connector **66** is on the outside of and surrounds part (an end) of second expandable stent **26.** An end of second expandable stent **26** emerges from tubular branch part **54** of stent connector **66.**

In Figure 5B, tubular trunk part **44** of stent connector **68** is on the outside of and surrounds first expandable stent **24.** First expandable stent **24** is substantially entirely contained within tubular trunk part **44** of stent connector **68.** Tubular branch part **54** of stent connector **68** is disposed inside the bore of and is substantially entirely contained within second expandable stent **26.**

In Figure 5C, tubular trunk part **44** of stent connector **70** is disposed entirely inside the bore of first expandable stent **24.** First expandable stent **24** is substantially entirely outside of tubular trunk part **44** of stent connector **68.** Tubular branch part **54** of stent connector **70** emerges from side opening **45** of first expandable stent **24.** Tubular branch part **54** of stent connector **70** entirely covers second expandable stent **26.** Second expandable stent **26** is substantially entirely contained within tubular branch part **54** of stent connector **70.**

In Figure 5D, tubular trunk part **44** of stent connector **72** is disposed inside the bore of first expandable stent **24.** The two ends of tubular trunk part **44** of stent connector **72** emerge from the ends of first expandable stent **24** and are folded over the respective end. Tubular branch part **54** of stent connector **72** emerges from side opening **45** of first expandable stent **24.** Tubular branch part **54** of stent connector **72** is disposed inside the bore of second expandable stent **26.** An end of tubular branch part **54** emerges from an end of second expandable stent **26** and is folded thereover. The folding allows for a simple to manufacture stent assembly of the present invention where stent connector **72** is securely attached to first expandable stent **24** and second expandable stent **26.**

In Figure 6A is depicted a stent assembly **78** of the present invention comprising stent connector **82.** In Figure 6B is depicted a stent assembly **80** of the present invention comprising stent connector **84.** Stent assemblies **78** and **80** include three expandable stents, a first expandable stent **24** that is destined for deployment in a trunk vessel and a second expandable stent **26** and a third expandable stent **32** that are both destined for deployment in branch vessels. Stent connectors **82** and **84** include three substantially tubular parts, a tubular trunk part **44** associated with first expandable stent **24,** and two tubular branch parts **54** branching from trunk part **44** and associated with second expandable stent **26** and third expandable stent 26. In such a way, any two stents of **24, 26** and **32** are associated at a desired distance but with virtually no other limitations as to the relative orientation of the two stents. Analogously to the discussed hereinabove, since tubular parts **44** and **54** of stent connectors 82 and **84** are disposed on the outside of stents **24, 26** and **32,** tubular parts **44** and **54** are held in place by tension and the attendant friction. The tubular parts expand together with a respective stent during expansion thereof. In Figure 6A, the two branch volumes (substantially defined by second expandable stent **26** and third expandable stent **32)** are in fluid communication with the trunk volume (substantially defined by first expandable stent **24)** through an end of first expandable stent **24** and therefore of the trunk volume. In Figure 6B, the two branch volumes (substantially defined by second expandable stent **26** and third expandable stent **32)** are in fluid communication with the trunk volume (substantially defined by first expandable stent **24)** through side openings **45** in first expandable stent **24** and therefore of the trunk volume.

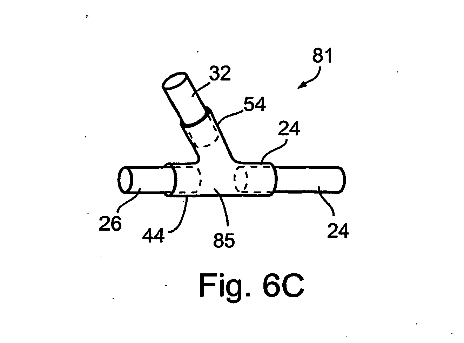

In Figure 6C is depicted a stent assembly **81** of the present invention comprising a stent connector **85.** Stent assembly **81** include three expandable stents, a first expandable stent **24** that is destined for deployment in a trunk vessel upstream of a bifurcation point **16,** a second expandable stent **26** destined for deployment in a trunk vessel downstream of a bifurcation point **16,** and a third expandable stent **32** that is destined for deployment in a branch vessel. Stent connector **85** is substantially similar to stent connector **66** depicted in Figure 5A and includes only two tubular parts, a tubular trunk part **44** associated with first expandable stent 24 and second expandable stent **26,** and a tubular branch part **54** branching from trunk part **44** and associated with third expandable stent **26.** In such a way, any two stents of **24, 26** and **32** are associated at a desired distance but with virtually no other limitations as to the relative orientation. Analogously to the discussed hereinabove, since tubular parts **44** and **54** of stent connector **85** are disposed on the outside of stents **24, 26** and **32,** tubular parts **44** and **54** are held in place by tension and the attendant friction. The tubular parts expand together with a respective stent during expansion thereof. In Figure 6C, the branch volume (substantially defined by third expandable stent **32**) is in fluid communication with the trunk volume (substantially defined by first expandable stent **24,** second expandable stent **26** and tubular trunk part **44**) through the gap between the upstream end of first expandable stent 24 and the downstream end of second expandable stent **26** and therefore of the trunk volume.

It is important to note that stent connectors **66, 68, 70, 72, 82, 84** and **85** depicted in Figures 5A, 5B, 5C, 5D, 6A, 6B and 6C are all substantially vessels comprising walls made of a flexible material provided with at least three ports. Stent connectors **66, 68, 70** and **72** are all vessels with three ports where a respective first expandable stent 24 is functionally associated with a first and a second of the three ports while a respective second expandable stent **26** is functionally associated with a third of the three ports. Further, each respective first expandable stent **24** of stent assemblies **58, 60, 62** and **64** includes a side opening **45** that is also functionally associated with the third of the three ports. Stent connectors **82** and **85** are vessels with three ports where a first of three ports is functionally associated with first expandable stent **24,** a second of three ports is functionally associated with second expandable stent **26** and a third of three ports is functionally associated with third expandable stent **32.** Stent connector **84** is a vessel with four ports where a first and second of four ports are functionally associated with first expandable stent **24,** a third of four ports is functionally associated with second expandable stent **26** and a fourth of four ports is functionally associated with third expandable stent **32.**

In stent connectors **66, 68, 70** and **72,** two ports (associated with first expandable stent **24**) and walls of the connector therebetween define a volume that is a trunk part **44** of the stent connector the volume substantially overlapping the trunk volume defined by the bore of a respective first expandable stent **24.** Together with the respective first expandable stent **24,** trunk part **44** of a stent connector defines a trunk implant, that is the part of the respective stent assembly destined for deployment in a trunk vessel.

In stent connectors **66, 68, 70** and **72** a third port (associated with second expandable stent **26**) is in communication with the trunk volume through tubular branch part **54** of the respective stent connector. Together with the respective second expandable stent **26,** tubular branch part **54** of a stent connector defines a branch implant, that is the part of the respective stent assembly destined for deployment in the lumen of a branch vessel.

In stent connector **85** of Figure 6C, two ports and walls of the connector therebetween define a volume that is a trunk part **44** of the stent connector, the volume substantially overlapping the trunk volume defined by the bores of first expandable stent **24** and second expandable stent **26.** Together with first expandable stent **24** and second expandable stent **26,** trunk part **44** of stent connector 85 defines a trunk implant, that is the part of the respective stent assembly destined for deployment in a trunk vessel. In stent connector **85** a third port (associated with third expandable stent **32**) is in communication with the trunk volume through tubular branch part **54.** Together with third expandable stent **32,** tubular branch part **54** of stent connector **85** defines a branch implant, that is the part of stent assembly **81** destined for deployment in the lumen of a branch vessel.

Stent assemblies of the present invention such as **58, 60, 62, 64, 78** and **80** and **81** are superior to prior art stent assemblies and, for certain uses, to stent assemblies of the present invention such as **22, 30, 34, 36, 46** and **48** due to the nature of the respective stent connector. In these cases, the stent connectors are vessels comprising walls that, when deployed in a bifurcated bodily vessel, support the vicinity of the bifurcation point of the bodily vessel. Such support prevents the collapse of the bodily vessel in the vicinity of the bifurcation point. Further, if during stent deployment and expansion the bodily vessel walls in the vicinity of the bifurcation become weakened, torn or otherwise damaged the respective stent connector acts to contain fluids flowing through the bodily vessel and prevents the fluids breaking through the thus damaged bodily vessel.

As depicted above, depending on the embodiment, tubular trunk parts **44** and tubular branch parts **54** of stent connectors of the present invention are disposed either on the outside of a respective stent or inside the bore of a stent.

Advantages of disposing tubular trunk parts **44** and tubular branch parts **54** inside the bore of stents include providing a smooth lumen wall allowing unrestricted and non-turbulent flow of fluids through the stent connector. Further, such an arrangement is useful for deployment in weakened or damaged vessels as the force applied by fluids flowing through such a stent connector to the tubular part is largely dissipated by the stents.

Advantages of disposing tubular trunk parts **44** and tubular branch parts **54** on the outside of stents include secure and even expansion, avoiding the use of sutures or other methods of securing the stent connector to the stents and allows for efficient relining of damaged vessel walls. Further, such an arrangement encases the area where any two stents meet, preventing pinching of bodily parts between the stents.

In Figures 3B, 4A, 4B, 5A, 5B, 5C and 5D first expandable stents **24** of stent assemblies **36, 46, 48, 58, 60, 62** and **64** are provided with a side opening **45.** In Figure 6B, first expandable stent **24** is provided with two side openings **45** (depicted in phantom). Side openings **45** and respective second expandable stents **26** are so positioned that the branch volume (substantially defined by second expandable stent **26**) is in fluid communication with the trunk volume (substantially defined by first expandable stent **24**) through side opening **45** and therefore from a side of the trunk volume. The use of a stent connector of the present invention ensures that a stent assembly is very bendable allowing changes in relative orientation of the component stents both in- and out-of-plane, in a very wide range of divergence angles. At the same time, the use of a stent connector of the present invention ensures that a given branch volume is always in unobstructed fluid communication with a trunk volume through a side opening 45 so that flow therethrough is unimpeded and not turbulent.

As noted hereinabove, it is preferable that the material from which a stent connector of the present invention is fashioned be flexible. In embodiments of the present invention it is also preferable that the stent connector be substantially elastic so as to better retain a desired shape during manipulation and deployment.

In the art stents with a jacket made of a material that is porous and permeable so as to allow cells to grow into and through the material, ultimately leading to the jacket becoming an integral part of the vessel in which deployed are known, see for example U.S. Patent 5,723,004. In embodiments of the present invention, the material from which a stent connector is fashioned is porous and/or permeable so as to allow tissue in-growth. A disadvantage of such permeability is that smooth muscle cells are known to grow in a disorganized and ultimately restenotic fashion through porous and permeable membranes.

In the art stents with a jacket made of a material that is substantially impervious to tissue proliferation therethrough are known, see for example PCT Patent Application PCT/IB98/01459 published as WO 99/15105 of the inventor. Such imperviousness is useful in preventing tissue buildup on and through the material and prevents the migration of smooth muscle cells. In embodiments of the present invention, the material from which a stent connector is fashioned is substantially impervious to tissue proliferation therethrough. Such embodiments are useful for providing a treated vessel with a smooth lining that is substantially impervious to restenosis.

In the art stents with a jacket made of a material that is impermeable to fluids so as to form a sealed vessel and thus avoid extravasation of fluids through the material are known. In embodiments of the present invention, the material from which a stent connector is made is impermeable to fluids.

Useful materials from which to fashion a stent connector of the present invention include synthetic or polymeric material including but not limited to polytetrafluoroethylene, urethane, elastomer, polyamide (e.g., Nylon) and polyester (e.g., Dacron).

Useful materials from which to fashion a stent connector of the present invention are also biological tissue including but not limited to autologous tissue, heterologous tissue, venous tissue, arterial tissue, serous tissue, pleura, peritoneum, pericardium and aortic leaflet. Generally suitable tissue types include but are not limited to equine, porcine, bovine or human tissue. It is often preferred that the tissue be thinned, that is after harvesting one or more layers of the harvested tissue are removed, e.g. by scraping, shaving, slicing or skiving (see U.S. Patent Nos. 6,468,300 and 6,254,627 of the inventor). In order to increase the toughness of the tissue, it is often advantageous to treat the tissue, for example with a glutaraldehyde or a phosphate solution, in order to cross-link collagen in the tissue.

Serous membranes are made of two strata of tissue. The serous stratum or layer of a serous membrane is a very smooth single layer of flattened, nucleated mesothelial cells united at their edges by a cement substance. The serous cells rest on a basement layer or stratum, a rough, strong fibrous layer. Serous membrane is one material that is strong, elastic and thin enough to be useful in implementing the teachings of the present invention. Thinned serous membrane is even more preferred as noted above and as taught in U.S. Patent Nos. 6,254,627 and 6,468,300 of the inventor. Not only is thinned serous membrane sufficiently strong, elastic and even thinner than serous membrane, thinned serous membrane also provides little resistance to radial expansion, making thinned serous membrane one of the few materials suitable for use in covering or jacketing self-expanding stents. Thus, in a preferred embodiment, the stent connector is substantially fashioned from a thinned serous membrane where a harvested serous membrane (peritoneum, pericardium or pleural tissue especially porcine, bovine, equine and human serous tissue) has been processed by removal of a layer of at least some of the basement tissue layer (and thus thinned), preferably removal of all the basement tissue, leaving only the serous tissue layer. In an embodiment of the present invention only a part of the basement tissue layer is removed. In an embodiment of the present invention, the thinned serous membrane is substantially serous tissue devoid of basement tissue. In an embodiment of the present invention, the material consists essentially of serous tissue.

When serous membrane or thinned serous membrane is used to implement the teachings of the present invention it is usually preferred to orient the membrane so that the smooth serous strata is facing the fluid flow to reduce turbulent flow.

A stent connector of a bifurcated stent assembly of the present invention is fashioned from one or more parts according to any of the methods with which one skilled in the art is acquainted.

In an embodiment of the present invention, tubular parts of a given stent connector are fashioned from a planar or substantially planar sheet of an appropriate membrane (synthetic or harvested), generally by rolling the sheet (with abutting or overlapping edges) and then fixing the tubular shape using any of the methods known in the art including but not limited to sutures, adhesives, bending members, clamps, glue, hooks, piercing members, staples, other applicable mechanical mean or combinations thereof). A stent connector made up of more than one tubular part is conveniently fashioned by making two separate tubes and conjoining the two tubes (using, for example, sutures, adhesives, bending members, clamps, glue, hooks, piercing members, staples, other applicable mechanical mean or combinations thereof).

In an embodiment of the present invention a stent connector is fashioned including seamless tubular parts. In an embodiment, a seamless tubular part of a stent connector of the present invention is fashioned from synthetic materials by methods including but not limited to, weaving synthetic fibers, molding a polymer or welding the seam of a rolled sheet, for instance using heat or an appropriate solvent. In an embodiment, a seamless tubular part of a stent connector of the present invention is fashioned from a section of harvested tubular biological tissue. In such a case a suitable tubular vessel, such as an appropriately sized autologous, homologous or heterologous artery or vein is identified, harvested, isolated and treated to prepare a seamless tubular stent connector component. Treatments include chemical or biological treatments, for example cross-linking or digestion, to increase flexibility or strength of the material of the tubular stent connector component. Treatments also include mechanical thinning to increase the flexibility and reduce thickness of the material of the stent connector component.

In embodiments of the present invention, a stent connector is made of two different materials. For example, the tubular stent connector wall is fashioned from a thin tube of serous tissue attached to a second tubular stent connector part made of a thicker material, for example, a harvested artery.

In an embodiment of the present invention a bifurcated stent connector is seamless. In an embodiment, a seamless bifurcated stent connector of the present invention is fashioned from synthetic materials by methods including but not limited to, weaving synthetic fibers, molding a polymer or welding separate components, for instance using heat or an appropriate solvent. In a preferred embodiment, a seamless bifurcated stent connector of the present invention is fashioned from a section of a bifurcated biological tissue. In such a case a suitable bifurcated vessel, such as an appropriately sized autologous, homologous or heterologous bifurcated artery or vein is identified, harvested, isolated and treated to prepare a seamless bifurcated stent connector. Treatments include chemical or biological treatments, for example cross-linking or digestion, to increase flexibility or strength of the material of the vessel and ultimately of the thus-fashioned stent connector. Treatments also include mechanical thinning to increase the flexibility and reduce thickness of the material of the vessel.

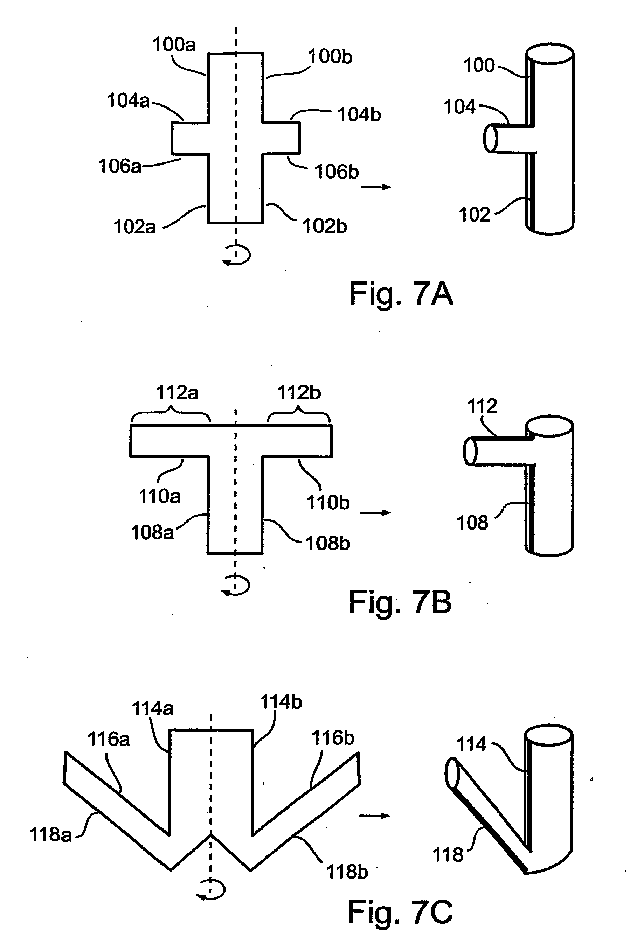

In an embodiment of the present invention, a bifurcated stent connector of the present invention is essentially fashioned from one sheet of planar or substantially planar sheet of an appropriate membrane (synthetic or harvested), rolled up into shape (with abutting or overlapping edges) and then fixed in shape using any of the methods known in the art including but not limited to sutures, adhesives, bending members, clamps, glue, hooks, piercing members, staples, other applicable mechanical mean or combinations thereof). Such an embodiment is preferred over a seamless bifurcated stent connector as the availability of suitable bifurcated vessels for harvesting is limited and limits the materials from which stent connector are fashioned. Such an embodiment is preferred over a bifurcated stent connector fashioned from two or more conjoined tubular components due to the reduced number of seams. Preferred shapes of sheets suitable for use in fashioning a bifurcated stent connector from one sheet of material generally include shapes having one C2 symmetry axis and at least one external angle of no greater than 90° and/or at least 7 sides. In Figures 7A-7G are depicted various suitable shapes rolled up and edges attached to fashion bifurcated stent connectors of the present invention.

In Figure 7A is depicted a cross-shaped sheet, rolled up as depicted by the arrow so that sides **100a** and **100b** are joined to form seam **100,** sides **102a** and **102b** are joined to form seam **102,** sides **104a** and **104b** are joined to form seam 104 and sides **106a** and **106b** are joined to form a non-depicted seam to fashion a bifurcated stent connector.

In Figure 7B is depicted a "T"-shaped sheet, rolled up as depicted by the arrow so that sides **108a** and **108b** are joined to form seam **108,** sides **110a** and **110b** are joined to form a non-depicted seam and sides **112a** and **112b** (delimited by the braces) are joined to form a seam **112** to fashion a bifurcated stent connector.

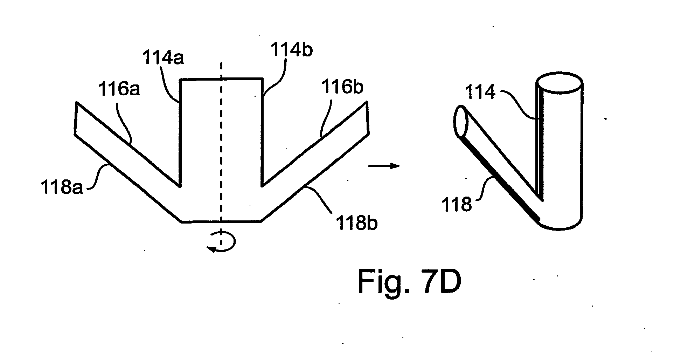

In Figures 7C and 7D are depicted "W"-shaped sheets, rolled up as depicted by the arrow so that sides **114a** and **114b** are joined to form seam **114,** sides **116a** and **116b** are joined to form a non-depicted seam and sides **118a** and **118b** are joined to form a seam **118** to fashion a bifurcated stent connector.

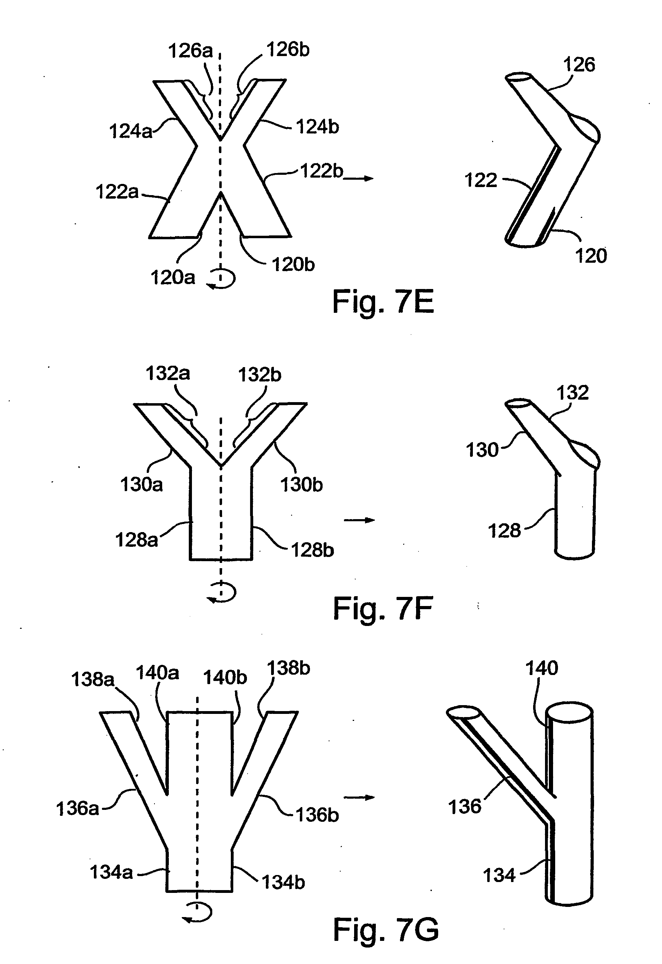

In Figure 7E is depicted an "X"-shaped sheet, rolled up as depicted by the arrow so that sides **120a** and **120b** are joined to form seam **120,** sides **122a** and **122b** are joined to form seam 122, sides **124a** and **124b** are joined to form a non-depicted seam, and sides **126a** and **126b** (delimited by the braces) are joined to form a seam **126** to fashion a bifurcated stent connector.

In Figure 7F is depicted a "Y"-shaped sheet, rolled up as depicted by the arrow so that sides **128a** and **128b** are joined to form seam **128,** sides **130a** and **130b** are joined to form seam **130** and sides **132a** and **132b** are joined to form seam **132** to fashion a bifurcated stent connector.

In Figure 7G is depicted a "Ψ"-shaped sheet, rolled up as depicted by the arrow so that sides **134a** and **134b** are joined to form seam **134,** sides **136a** and **136b** are joined to form seam **136,** sides **138a** and **138b** are joined to form a non-depicted seam and sides **140a** and **140b** are joined to form seam **140** to fashion a bifurcated stent connector.

Any suitable method known in the art may be used to conjoin any two components or to attach ends of a single piece of material to fashion a stent connector of the present invention. Such methods include but are not limited to sutures, adhesives, clamps, glue, hooks, piercing members, staples, laser welding other applicable mechanical mean or combinations thereof. In embodiments of the present invention two components or two ends of a single piece of material are overlapped and joined. An overlapping joint is leak resistant and strong due to increased surface area of the seam. In embodiments of the present invention two components or two ends of a single piece of material are abutted and joined. An abutting joint is thinner than an overlapping joint.

Once a stent connector of the desired topography is made, assembly of a bifurcated stent assembly is straightforward for one skilled in the art upon perusal of the disclosure herein. Generally, a stent is selected and contacted with an appropriate part of the stent connector. When a stent is surrounded by a substantially tubular part of a stent connector, the stent is generally threaded through the tubular part to the desired extent. As noted hereinabove, a given stent is secured to an appropriate part of a respective stent connector with sutures or by a combination of tension and concomitant friction of a tubular stent connector section surrounding a stent. That said, the stent is secured to a respective stent connector using any of the methods known in the art, including but not limited to adhesives, bending members, clamps, glue, hooks, piercing members, staples, other applicable mechanical mean or combinations thereof).

One of the design features of a stent connector comprising a tubular part is the length of the tubular part relative to a stent with which the tubular part is associated, that is to say, what part of the outer surface of a stent is covered by a respective tubular part (when the tubular part is disposed outside the stent) or what part of the inside surface of a stent is in contact with a respective tubular part (when the tubular part is disposed inside the stent). As noted hereinabove, in embodiments of the present invention a tubular part is substantially as long or even longer than a stent with which that tubular part is associated. In embodiments of the present invention the tubular part is substantially shorter than an associated stent, for example up to 50% of the length of an associated stent, up to 40% of the length of an associated stent, up to 30% of the length of an associated stent or even up to 20% of the length of an associated stent.

Generally the walls of a stent connector of a bifurcated stent assembly of the present invention are as thin as possible yet must be elastic enough and strong enough to be useful, that is to allow navigation and deployment of the bifurcated stent assembly including movement of the component stents of the bifurcated stent assembly without tearing. Clearly, the nature of the material from which a given stent connector is made determines in part the thickness of that stent connector. That said, a stent connector of a bifurcated stent assembly of the present invention preferably has walls that are not thicker than about 0.75 mm, not thicker than about 0.45 mm, not thicker than about 0.25 mm, not thicker than about 0.20 mm, and even not thicker than about 0.05 mm. A useful material from which to fashion a stent connector of a bifurcated stent assembly of the present invention having the appropriate thickness yet also being smooth to reduce the chances of restenosis, being sufficiently strong and flexible is thinned serous membrane as discussed above, especially bovine pericardium as disclosed in PCT Patent application No. PCT/IB98/01459 published as WO 99/15105 of the inventor.

Generally any type of stent known in the art is useful as a component of a bifurcated stent assembly of the present invention. Such stents include but are not limited to stents marketed by affiliates (e.g., Cordis, Centocor) of Johnson & Johnson, Guidant (Indianapolis, Indiana, USA), Medtronic (Minneapolis, Minnesota, USA), Medinol (Tel Aviv, Israel), Cook Inc. (Bloomington, Indiana, USA) and PM Devices Inc. (Richmond, British Columbia, Canada). In embodiments of the stent assembly of the present invention, a first expandable stent, a second expandable stent (and third or more expandable stents if present) are of substantially similar or identical dimensions, especially length, expanded diameter and/or unexpanded diameter.

In embodiments of the present invention a first expandable stent, a second expandable stent (and third or more expandable stents if present) are of substantially different dimensions, especially length, expanded diameter and/or unexpanded diameter. In cases where the first expandable stent, the second expandable stent (and third or more expandable stents if present) are of different dimensions, generally the first expandable stent is larger (especially of larger expanded and/or unexpanded diameter) as it is generally the first expandable stent that is destined to be deployed in a trunk vessel rather than a smaller-bored branch vessel.

In embodiments of the present invention, the diameter (expanded or unexpanded) of the first expandable stent is substantially similar to the respective diameter of the second expandable stent (and third or more expandable stents if present).

In embodiments of the present invention, the diameter (expanded or unexpanded) of the first expandable stent is larger than the respective diameter of the second expandable stent (and third or more expandable stents if present).

In embodiments of a bifurcated stent assembly of the present invention a first expandable stent has a length of up to about 80 mm, up to about 65 mm, or even up to about 50 mm.

In embodiments of a bifurcated stent assembly of the present invention a first expandable stent has a length of greater than about 5 mm, greater than about 7 mm, or even greater than about 10 mm.

In embodiments of a bifurcated stent assembly of the present invention a second expandable stent has a length of up to about 80 mm, up to about 65 mm, or even up to about 50 mm.

In embodiments of a bifurcated stent assembly of the present invention a second expandable stent has a length of greater than about 5 mm, greater than about 7 mm, or even greater than about 10 mm.

Two important parameters used when selecting a stent for use are the expanded and unexpanded diameters of the stent.

Generally it is important that the unexpanded diameter of a stent be as small as possible to ease navigation through the bodily lumen to the deployment location. That said, the unexpanded diameter must be large enough to allow threading of the stent onto a deployment catheter and, if necessary, a stent-expanding device such as a stent-expanding balloon. Although there may be some variation in the unexpanded diameter of even two identical stents depending on how the two stents are used, herein by unexpanded diameter is intended the outer diameter of an expandable stent when crimped to the greatest extent onto a delivery catheter for deployment.

In embodiments of a bifurcated stent assembly of the present invention a first expandable stent has an unexpanded diameter as defined above of at least about 0.5 mm, at least about 1 mm, and even at least about 2 mm. In embodiments of a bifurcated stent assembly of the present invention a second expandable stent (or third or more expandable stents if present) has an unexpanded diameter as defined above of at least about 0.5 mm, at least about 1 mm, and even at least about 2 mm.

Generally, any given stent has a wide range of expanded diameters larger than a respective unexpanded diameter. The expanded diameter of a stent subsequent to deployment is determined by the user of the stent according to medical criteria including the natural size of the lumen of the vessel in which the stent is deployed. That said, self-expanding stents are characterized by a specific maximal expansion that is the maximal diameter of the stent when the stent is free from externally applied forces. Most not self-expanding stents are also characterized by a maximal expansion that is the greatest extent to which the stent is expandable without comprising the structural integrity thereof.

In embodiments of a bifurcated stent assembly of the present invention a first expandable stent has a maximal expanded diameter as defined above of up to about 30 mm, up to about 8 mm, up to about 6 mm, and even up to about 5 mm.

In embodiments of a bifurcated stent assembly of the present invention a second expandable stent (and third or more expandable stents if present) has a maximal expanded diameter as defined above of up to about 30 mm up, to about 8 mm, up to about 6 mm, and even up to about 5 mm.

The first expandable stent and a second expandable stent of a bifurcated stent assembly of the present invention are relatively close together to ensure maximal support of a treated vessel when deployed. Thus, in embodiments of the present invention the distance between the first expandable stent and the second expandable stent is no greater than about four, no greater than about three, no greater than about two, no greater than about one and even no greater than about half of an unexpanded diameter of the second expandable stent.

It is known in the art to deploy a stent provided with a stent jacket. The stent jacket is generally a tubular membrane placed on the outer surface of the stent although internal stent jackets, substantially tubular membranes held within the bore of the stent, are known (see, for example, U.S. Patent Nos. 6,254,627 and 6,699,277). The stent jacket provides a smooth lumen for the treated vessel, reduces turbulent flow through the treated vessel, and provides structural reinforcement. In embodiments of the present invention, one or more of the component stents of a bifurcated stent assembly are jacketed. For example, any of the different stent jackets known to one skilled in the art are useful for jacketing one, some or all component stents of a stent assembly of the present invention.

It is known in the art to deploy a coated stent. Many different coatings are known in the art, for example, anti-thrombogenic coatings, anti-angiogenic coatings, anti-coagulant coatings and active pharmaceutical ingredient delivering coatings. Any of the different stent coatings known to one skilled in the art are useful for coating one, some or all component stents of a stent assembly of the present invention.

Deployment of a stent assembly of the present invention is preferably performed according to the methods known in the art, for example as described in U.S. Patent 5,723,004 or PCT Patent application PCT/IB98/00496 published as WO 99/15103 of the inventor. Deployment of a stent assembly **58** depicted in Figure 5A into a vessel **10** as depicted in Figure 1A is discussed in detail hereinbelow, where first expandable stent **24** and second expandable stent **26** are non-self expanding.

In brief, according to one method of deploying a stent assembly, two guidewires are navigated through the body, up through a trunk vessel to be treated, and past bifurcation point **16,** a first guidewire in trunk vessel **12** and a second guidewire into branch vessel **14.** Thereafter, stent assembly **58** in the unexpanded state is mounted on a delivery system including one delivery catheter for each stent of stent assembly **58,** preferably including two balloon catheters for each stent of stent assembly **58,** or a balloon catheter for first expandable (trunk) stent 24 and a transfer catheter for second expandable (branch) stent **26.** The delivery catheter on which first expandable stent **24** is mounted is positioned over the first guidewire and the delivery catheter on which second expandable stent **26** is mounted is positioned over the second guidewire. The two delivery catheters are advanced along the respective guidewires and thus navigated through the body to the proper location in vessel **10,** first expandable stent **24** between lines A-A and B-B and across bifurcation point **16** and second expandable stent **26** into branch part **14.** Stent connector **66** is placed snugly against the bifurcation as stent connector **66** prevents any possibility of pushing stent assembly 58 too far and damaging vessel **10.** Further, stent connector **66** allows each component stent, **24** and **26** to bend and independently move relative to the other, without leading to any distortion, bending or buckling in the other stent. Second expandable stent **26** is pushed into branch part **14** until resistance of stent connector **66** is felt. In such a way it is ensured that second expandable stent **26** is not too close to bifurcation point **16** and does not protrude into trunk vessel **12.** At the same time, stent connector **66** prevents second expandable stent **26** from being pushed too far into branch part **14.** Once properly located first expandable stent **24** and second expandable stent **26** are expanded, whether simultaneously or serially, each to an expanded state of a desired size. Expansion of a stent generally occurs by positioning an inflation device (such as the balloon of a balloon catheter) inside the bore of a respective stent, inflating the balloon to a desired extent so as to expand the stent to a desired extent, and susequently deflating the balloon for withdrawal from the body.

In an example where a delivery device includes a balloon catheter for each stent, the balloon is generally already properly located within the bore of a respective stent. First expandable stent **24** and second expandable stent **26** are either simultaneously or serially expanded to a desired extent by inflation of the respective balloons. The balloons are deflated and the delivery catheters withdrawn from the body.

In an example where a delivery device already includes a balloon catheter on which first expandable stent **24** is mounted and a transfer catheter (without a balloon) on which second expandable stent **26** is mounted, the balloon is generally already properly located within the bore of first expandable stent **24.** In a first step, the balloon is inflated to expand first expandable stent **24** to a desired extent and deflated. In a second step, the transfer catheter is withdrawn and a replacement balloon catheter advanced over the second guidewire until the balloon thereon is properly positioned inside the bore of second expandable stent **26.** The balloon of the replacement balloon catheter is inflated to expand second expandable stent 26 to a desired extent and deflated. The two catheters are withdrawn from the body.

Deployment of other stent assemblies in vessels such as **10** depicted in Figure 1A or **18** depicted in Figure 1B, or when first expandable stent **24** or second expandable stent 26 or both are self-expanding is understood by one skilled in the art upon perusal of the description herein.

Although described with respect to treating bifurcated vessels of the cardiovascular system, and especially bifurcated arteries, the teachings of the present invention are generally applicable to many different cardiovascular and non-cardiovascular applications. Specific cardiovascular applications include but are not limited to the deployment of a bifurcated stent of the present invention in ectatic arteries and ectatic arteries containing an obstructive lesion, aneurismatic arteries, saphenous vein grafts and native arteries, coronary perforation, coronary fistula, ostial coronary lesions, aortic abdominal aneurysm and other aneurismatic peripheral arteries, transjugular intrahepatic portal shunt, percutaneous transluminal angioplasty, fistula closing and neuro interventions (such as aneurysms and arterial-venous malformations), small vessel intraluminal grafting, and ostial renal artery lesions. Additional non-cardiovascular applications include but are not limited to urological, gastroenterological, respiratory and neurological applications.

## Claims

1. A method for bendably associating a first expandable stent (24) with a second expandable stent (26) for deployment in a bifurcated vessel comprising using a flexible membrane as a stent connector (28, 38, 40, 50, 52, 66, 68, 70, 72, 82, 84, 85) to connect a second expandable stent (26) to a first expandable stent (24) so that the bore of said first expandable stent (24) defines an elongated trunk volume and the bore of said second expandable stent (26) defines an elongated branch volume branching from said trunk volume wherein said stents are substantially devoid of direct physical association, **characterised in that**
said stent connector (28, 38, 40, 50, 52, 66, 68, 70, 72, 82, 84, 85) includes a first substantially tubular trunk part (44) functionally associated with said first expandable stent (24) and a second substantially tubular branch part (54) functionally associated with said second expandable stent (26), said branch part (54) branching from said trunk part (44); and
wherein the distance between said first expandable stent (24) and said second expandable stent (26) is no greater than about four times the unexpanded diameter of said second expandable stent (26).

2. A bifurcated stent assembly (22, 30, 34, 36, 46, 48, 58, 60, 62, 64, 78, 80, 81), comprising:
a) a flexible membrane as a stent connector (28, 38, 40, 50, 52, 66, 68, 70, 72, 82, 84, 85) ;
b) a first expandable stent (24) functionally associated with said stent connector (28, 38, 40, 50, 52, 66, 68, 70, 72, 82, 84, 85), wherein the bore of said first expandable stent (24) defines an elongated trunk volume; and
c) a second expandable stent (26) functionally associated with said stent connector (28, 38, 40, 50, 52, 66, 68, 70, 72, 82, 84, 85), wherein the bore of said second expandable stent (26) defines an elongated branch volume branching from said trunk volume wherein said stents are substantially devoid of direct physical association **characterised in that**
said stent connector (28, 38, 40, 50, 52, 66, 68, 70, 72, 82, 84, 85) includes a first substantially tubular trunk part (44) functionally associated with said first expandable stent (24) and a second substantially tubular branch part (54) functionally associated with said second expandable stent (26), said branch part (54) branching from said trunk part (44); and
wherein the distance between said first expandable stent (24) and said second expandable stent (26) is no greater than about four times the unexpanded diameter of said second expandable stent (26).

3. The method or the bifurcated stent assembly (22, 30, 34, 36, 46, 48, 58, 60, 62, 64, 78, 80, 81) of any of claims 1 or 2, wherein said first expandable stent (24) is provided with a side opening (45) through which said branch volume is in fluid communication with said trunk volume.

4. The method or the bifurcated stent assembly (22, 30, 34, 36, 46, 48, 58, 60, 62, 64, 78, 80, 81) of any of claims 1 or 2, wherein said branch volume is in fluid communication with said trunk volume from an end of said trunk volume.

5. The method or the bifurcated stent assembly (22, 30, 34, 36, 46, 48, 58, 60, 62, 64, 78, 80, 81) of any of claims 1 to 4, wherein the distance between said first expandable stent (24) and said second expandable stent (26) is no greater than about three times the unexpanded diameter of said second expandable stent (26).

6. The method or bifurcated stent assembly (22, 30, 34, 36, 46, 48, 58, 60, 62, 64, 78, 80, 81) of any of claims 1 to 5, further comprising: a third expandable stent (32) functionally associated with said stent connector (28, 38, 40, 50, 52, 66, 68, 70, 72, 82, 84, 85),
wherein the bore of said third expandable stent (32) defines an elongated second branch volume branching from said trunk volume, and wherein said stent connector (28, 38, 40, 50, 52, 66, 68, 70, 72, 82, 84, 85) includes a substantially tubular branch part (54) functionally associated with said third expandable stent (32).

7. The method or bifurcated stent assembly (22, 30, 34, 36, 46, 48, 58, 60, 62, 64, 78, 80, 81) of claim 6, wherein said second branch volume is in fluid communication with said trunk volume from a side of said trunk volume.

8. The method of bifurcated stent assembly (22, 30, 34, 36, 46, 48, 58, 60, 62, 64, 78, 80, 81) of claim 6, wherein said second branch volume is in fluid communication with said trunk volume from an end of said trunk volume.

9. The method or bifurcated stent assembly (22, 30, 34, 36, 46, 48, 58, 60, 62, 64, 78, 80, 81) of any of claims 1 to 8, wherein said stent connector (28, 38, 40, 50, 52, 66, 68, 70, 72, 82, 84, 85) is substantially a vessel comprising walls of a flexible material and at least three ports, a first port, a second port and a third port, and, wherein said first expandable stent (24) is functionally associated with said first port and said second expandable stent (26) is functionally associated with said third port.

10. The method or bifurcated stent assembly (22, 30, 34, 36, 46, 48, 58, 60, 62, 64, 78, 80, 81) of claim 9, further comprising a third expandable stent (32) functionally associated with said second port.

## Patentansprüche

1. Verfahren zum biegbaren Verbinden eines ersten ausdehnbaren Stents (24) mit einem zweiten ausdehnbaren Stent (26) zum Einsatz in einem gegabelten Gefäß umfassend ein Verwenden einer flexiblen Membran als Stentanschluss (28, 38, 40, 50, 52, 66, 68, 70, 72, 82, 84, 85) um einen zweiten ausdehnbaren Stent (26) mit einem ersten ausdehnbaren Stent (24) zu verbinden, so dass die Bohrung des ersten ausdehnbaren Stents (24) ein längliches Stammvolumen definiert und die Bohrung des zweiten ausdehnbaren Stents (26) ein längliches Zweigvolumen, das sich von dem Stammvolumen abzweigt, wobei die Stents im Wesentlichen frei von einer direkten körperlichen Verbindung miteinander sind, **dadurch gekennzeichnet, dass**
der Stentanschluss (28, 38, 40, 50, 52, 66, 68, 70, 72, 82, 84, 85) einen ersten im Wesentlichen röhrenförmigen Stammteil (44) beinhaltet, der funktionell mit dem ersten ausdehnbaren Stent (24) verbunden ist, und einen zweiten im Wesentlichen röhrenförmigen Zweigteil (54), der funktionell mit dem zweiten ausdehnbaren Stent (26) verbunden ist, wobei sich der Zweigteil (54) von dem Stammteil (44) abzweigt, und
wobei die Entfernung zwischen dem ersten ausdehnbaren Stent (24) und dem zweiten ausdehnbaren Stent (26) nicht größer ist als ungefähr viermal der Durchmesser des zweiten ausdehnbaren Stents (26) in nicht ausgedehntem Zustand.

2. Gegabelte Stentanordnung (22, 30, 34, 36, 46, 48, 58, 60, 62, 64, 78, 80, 81) umfassend:
a) eine flexible Membran als Stentanschluss (28, 38, 40, 50, 52, 66, 68, 70, 72, 82, 84, 85),
b) einen mit dem Stentanschluss (28, 38, 40, 50, 52, 66, 68, 70, 72, 82, 84, 85) funktionell verbundenen ersten ausdehnbaren Stent (24), worin die Bohrung des ersten ausdehnbaren Stents (24) ein längliches Stammvolumen definiert, und
c) einen mit dem Stentanschluss (28, 38, 40, 50, 52, 66, 68, 70, 72, 82, 84, 85) funktionell verbundenen zweiten ausdehnbaren Stent (26), worin die Bohrung des zweiten ausdehnbaren Stents (26) ein längliches Zweigvolumen definiert, das sich von dem Stammvolumen abzweigt, worin die Stents im Wesentlichen frei von einer direkten körperlichen Verbindung miteinander sind, **dadurch gekennzeichnet, dass**
der Stentanschluss (28, 38, 40, 50, 52, 66, 68, 70, 72, 82, 84, 85) einen ersten im Wesentlichen röhrenförmigen Stammteil (44) beinhaltet, der funktionell mit dem ersten ausdehnbaren Stent (24) verbunden ist, und einen zweiten im Wesentlichen röhrenförmigen Zweigteil (54), der funktionell mit dem zweiten ausdehnbaren Stent (26) verbunden ist, worin sich der Zweigteil (54) von dem Stammteil (44) abzweigt, und
worin die Entfernung zwischen dem ersten ausdehnbaren Stent (24) und dem zweiten ausdehnbaren Stent (26) nicht größer ist als ungefähr viermal der Durchmesser des zweiten ausdehnbaren Stents (26) in nicht ausgedehntem Zustand.

3. Verfahren oder gegabelte Stentanordnung (22, 30, 34, 36, 46, 48, 58, 60, 62, 64, 78, 80, 81) nach einem der Ansprüche 1 und 2, worin der erste ausdehnbaren Stent (24) mit einer seitlichen Öffnung (45) bereitgestellt ist, durch die das Zweigvolumen in fluider Verbindung mit dem Stammvolumen steht.

4. Verfahren oder gegabelte Stentanordnung (22, 30, 34, 36, 46, 48, 58, 60, 62, 64, 78, 80, 81) nach einem der Ansprüche 1 und 2, worin das Zweigvolumen mit dem Stammvolumen von einem Ende des Stammvolumens in fluider Verbindung steht

5. Verfahren oder gegabelte Stentanordnung (22, 30, 34, 36, 46, 48, 58, 60, 62, 64, 78, 80, 81) nach einem der Ansprüche 1 bis 4, worin die Entfernung zwischen dem ersten ausdehnbaren Stent (24) und dem zweiten ausdehnbaren Stent (26) nicht größer ist als ungefähr dreimal der Durchmesser des zweiten ausdehnbaren Stents (26) in nicht ausgedehntem Zustand.

6. Verfahren oder gegabelte Stentanordnung (22, 30, 34, 36, 46, 48, 58, 60, 62, 64, 78, 80, 81) nach einem der Ansprüche 1 bis 5, ferner einen dritten ausdehnbaren Stent (32) umfassend, der funktionell mit dem Stentanschluss (28, 38, 40, 50, 52, 66, 68, 70, 72, 82, 84, 85) verbunden ist, worin die Bohrung des dritten ausdehnbaren Stents (32) ein längliches zweites Zweigvolumen definiert, das von dem Stammvolumen abzweigt, und worin der Stentanschluss (28, 38, 40, 50, 52, 66, 68, 70, 72, 82, 84, 85) einen im Wesentlichen röhrenförmigen Zweigteil (54) beinhaltet, der mit dem dritten ausdehnbaren Stent (32) funktionell verbunden ist.

7. Verfahren oder gegabelte Stentanordnung (22, 30, 34, 36, 46, 48, 58, 60, 62, 64, 78, 80, 81) nach Anspruch 6, worin das zweite Zweigvolumen mit dem Stammvolumen von einer Seite des Stammvolumens in fluider Verbindung steht.

8. Verfahren oder gegabelte Stentanordnung (22, 30, 34, 36, 46, 48, 58, 60, 62, 64, 78, 80, 81) nach Anspruch 6, worin das zweite Zweigvolumen mit dem Stammvolumen von einem Ende des Stammvolumens in fluider Verbindung steht.

9. Verfahren oder gegabelte Stentanordnung (22, 30, 34, 36, 46, 48, 58, 60, 62, 64, 78, 80, 81) nach einem der Ansprüche 1 bis 8, worin der Stentanschluss (28, 38, 40, 50, 52, 66, 68, 70, 72, 82, 84, 85) im Wesentlichen ein Gefäß ist, das Wandungen aus einem flexiblen Material umfasst sowie wenigstens drei Anschlüsse, einen ersten Anschluss, einen zweiten Anschluss und einen dritten Anschluss, und worin der erste ausdehnbare Stent (24) mit dem ersten Anschluss funktionell verbunden ist und der zweite ausdehnbare Stent (26) mit dem dritten Anschluss funktionell verbunden ist.

10. Verfahren oder gegabelte Stentanordnung (22, 30, 34, 36, 46, 48, 58, 60, 62, 64, 78, 80, 81) nach Anspruch 9, ferner einen dritten ausdehnbaren Stent (32) umfassend, der funktionell mit dem zweiten Anschluss verbunden ist.

## Revendications

1. Procédé d'association, de manière à pouvoir plier, d'une première endoprothèse expansible (24) à une deuxième endoprothèse expansible (26) pour la mise en place dans un vaisseau bifurqué, comprenant l'utilisation d'une membrane souple comme raccord d'endoprothèses (28, 38, 40, 50, 52, 66, 68, 70, 72, 82, 84, 85) pour raccorder une deuxième endoprothèse expansible (26) à une première endoprothèse expansible (24) de manière à ce que le trou de ladite première endoprothèse expansible (24) définit un volume de tronc allongé et le trou de ladite deuxième endoprothèse expansible (26) définit un volume de ramification allongé se ramifiant à partir dudit volume de tronc, lesdites endoprothèses étant substantiellement dépourvues d'association physique directe, **caractérisé en ce que**
ledit raccord d'endoprothèse (28, 38, 40, 50, 52, 66, 68, 70, 72, 82, 84, 85) comprend une première partie de tronc substantiellement tubulaire (44) associée de manière fonctionnelle à ladite première endoprothèse expansible (24) et une deuxième partie de ramification substantiellement tubulaire (54) associée de manière fonctionnelle à ladite deuxième endoprothèse expansible (26), ladite partie de ramification (54) se ramifiant à partir de ladite partie de tronc (44) ; et
la distance entre ladite première endoprothèse expansible (24) et ladite deuxième endoprothèse expansible (26) n'étant pas supérieure à environ quatre fois le diamètre non expansé de ladite deuxième endoprothèse expansible (26).

2. Ensemble d'endoprothèse bifurquée (22, 30, 34, 36, 46, 48, 58, 60, 62, 64, 78, 80, 81), comprenant:
a) une membrane souple comme raccord d'endoprothèse (28, 38, 40, 50, 52, 66, 68, 70, 72, 82, 84, 85) ;
b) une première endoprothèse expansible (24) associée de manière fonctionnelle audit raccord d'endoprothèse (28, 38, 40, 50, 52, 66, 68, 70, 72, 82, 84, 85), le trou de ladite première endoprothèse expansible (24) définissant un vo-lume de tronc allongé ; et
c) une deuxième endoprothèse expansible (26) associée de manière fonctionnelle audit raccord d'endoprothèse (28, 38, 40, 50, 52, 66, 68, 70, 72, 82, 84, 85), le trou de ladite deuxième endoprothèse expansible (26) définissant un volume de ramification allongé se ramifiant à partir dudit volume de tronc, lesdites endoprothèses étant substantiellement dépourvues d'association physique directe, **caractérisé en ce que**
ledit raccord d'endoprothèse (28, 38, 40, 50, 52, 66, 68, 70, 72, 82, 84, 85) comprend une première partie de tronc substantiellement tubulaire (44) associée de manière fonctionnelle à ladite première endoprothèse expansible (24) et une deuxième partie de ramification substantiellement tubulaire (54) associée de manière fonctionnelle à ladite deuxième endoprothèse expansible (26), ladite partie de ramification (54) se ramifiant à partir de ladite partie de tronc (44) ; et
la distance entre ladite première endoprothèse expansible (24) et ladite deuxième endoprothèse expansible (26) n'étant pas supérieure à environ quatre fois le diamètre non expansé de ladite deuxième endoprothèse expansible (26).

3. Procédé ou ensemble d'endoprothèse bifurquée (22, 30, 34, 36, 46, 48, 58, 60, 62, 64, 78, 80, 81) selon l'une quelconque des revendications 1 ou 2, ladite première endoprothèse expansible (24) étant dotée d'une ouverture latérale (45) à travers laquelle ledit volume de ramification est en communication fluidique avec ledit volume de tronc.

4. Procédé ou ensemble d'endoprothèse bifurquée (22, 30, 34, 36, 46, 48, 58, 60, 62, 64, 78, 80, 81) selon l'une quelconque des revendications 1 ou 2, ledit volume de ramification étant en communication fluidique avec ledit volume de tronc à partir d'une extrémité dudit volume de tronc.

5. Procédé ou ensemble d'endoprothèse bifurquée (22, 30, 34, 36, 46, 48, 58, 60, 62, 64, 78, 80, 81) selon l'une quelconque des revendications 1 à 4, la distance entre ladite première endoprothèse expansible (24) et ladite deuxième endoprothèse expansible (26) n'étant pas supérieure à environ trois fois le diamètre non expansé de ladite deuxième endoprothèse expansible (26).

6. Procédé ou ensemble d'endoprothèse bifurquée (22, 30, 34, 36, 46, 48, 58, 60, 62, 64, 78, 80, 81) selon l'une quelconque des revendications 1 à 5, comprenant en outre : une troisième endoprothèse expansible (32) associée de manière fonctionnelle audit raccord d'endoprothèse (28, 38, 40, 50, 52, 66, 68, 70, 72, 82, 84, 85),
le trou de ladite troisième endoprothèse expansible (32) définissant un volume de deuxième ramification allongé se ramifiant à partir dudit volume de tronc et ledit raccord d'endoprothèse (28, 38, 40, 50, 52, 66, 68, 70, 72, 82, 84, 85) comportant une partie de ramification substantiellement tubulaire (54) associée de manière fonctionnelle à ladite troisième endoprothèse expansible (32).

7. Procédé ou ensemble d'endoprothèse bifurquée (22, 30, 34, 36, 46, 48, 58, 60, 62, 64, 78, 80, 81) selon la revendication 6, ledit volume de deuxième ramification étant en communication fluidique avec ledit volume de tronc à partir d'un côté dudit volume de tronc.

8. Procédé ou ensemble d'endoprothèse bifurquée (22, 30, 34, 36, 46, 48, 58, 60, 62, 64, 78, 80, 81) selon la revendication 6, ledit volume de deuxième ramification étant en communication fluidique avec ledit volume de tronc à partir d'une extrémité dudit volume de tronc.

9. Procédé ou ensemble d'endoprothèse bifurquée (22, 30, 34, 36, 46, 48, 58, 60, 62, 64, 78, 80, 81) selon l'une quelconque des revendications 1 à 8, ledit raccord d'endoprothèse (28, 38, 40, 50, 52, 66, 68, 70, 72, 82, 84, 85) consistant substantiellement en un conduit comprenant des parois en un matériau souple et au moins trois orifices, un premier orifice, un deuxième orifice et un troisième orifice et ladite première endoprothèse expansible (24) étant associée de manière fonctionnelle audit premier orifice et ladite deuxième endoprothèse expansible (26) étant associée de manière fonctionnelle audit troisième orifice.

10. Procédé ou ensemble d'endoprothèse bifurquée (22, 30, 34, 36, 46, 48, 58, 60, 62, 64, 78, 80, 81) selon la revendication 9, comprenant en outre une troisième endoprothèse expansible (32), associée de manière fonctionnelle audit deuxième orifice.
